# EUROPEAN PATENT SPECIFICATION

(11) **EP 2 371 402 B1**
(45) Date of publication and mention of the grant of the patent: **15.01.2014**
(21) Application number: 11158655.8
(22) Date of filing: 17.03.2011
(51) Int. Cl.: A61L 31/10, A61L 31/14, A61M 25/09

(54) **Guidewire coating presenting a crosslinking gradient**
Führungsdrahtsbeschichtung mit Vernetzungsgradient
Revêtement de fil-guide présentant un gradient de réticulation

(30) Priority: 31.03.2010 JP 2010080059
(43) Date of publication of application: 05.10.2011
(73) Proprietor: Asahi Intecc Co., Ltd., Nagoya-shi, Aichi 463-0024 (JP)
(72) Inventor: Satozaki, Junji, Nagoya-shi Aichi 463-0024 (JP)
(74) Representative: Winter, Brandl, Fürniss, Hübner, Röss, Kaiser, Polte - Partnerschaft

(56) References cited:
- EP-A2- 0 109 943
- WO-A2-98/55172
- US-A- 5 772 609

## Description

### Technical Field

The present invention relates to a guidewire forming a lubricating surface.

### Background Art

Conventionally, as for a guidewire that guides a catheter or the like used by being inserted in a tubular organ such as a vessel, a digestive tract, or a ureter or an intracorporeal tissue for treatment or examination, several kinds each provided on its surface with a hydrophilic coating agent to reduce a friction with the intracorporeal tissue are proposed.

For example, a hydrogel described in Japanese Patent Application Laid-Open No. 2004-215709 is formed by irradiating, with radiation, a solution containing a polymer having high biocompatibility and a polymeric compound cross-linked by radiation.

Also, in a guidewire described in Japanese Patent Application Laid-Open No. 2008-125913, a coating material containing a cross-linked polymer with 2-acrylamide-2-methylpropanesulfonic acid as a first monomer component is used as a material for a coating layer coating at least a part of an outer circumference of a wire main body.

The patent US 5772609 describes a guidewire presenting a gradient of flexibility along the length of the medical device in order to prevent the coating to crack.

### Summary of Invention

However, in the case of merely using the polymer having high biocompatibility or the cross-linked hydrophilic polymer as the material for the hydrophilic coating agent as described in Japanese Patent Application Laid-Open No. 2004-215709 or 2008-125913, the entire coating layer to be formed will be hardened. In this case, flexibility of the guidewire is impaired, and thus the guidewire may perforate a vessel or the like at the time of operating the guidewire. Also, in a case of bending such a guidewire whose entire layer has been hardened, cracks may be generated at the coating layer. In this case, the coating layer may be peeled due to these cracks. Further, in the cross-linking reaction of the hydrophilic coating agent, hydrophilic functional groups are used. Thus, in a case where the hydrophilic coating agent is merely cross-linked evenly, the amount of the hydrophilic functional groups existing on the surface of the hydrophilic coating agent will decrease. This degrades lubricity of the guidewire, which causes degradation in operability of the guidewire.

The present invention has been made to solve the foregoing technical problems, and an object of the present invention is to provide a guidewire preventing cracks from being generated at a hydrophilic coating agent and excellent in lubricity, by securing flexibility of the hydrophilic coating agent on its surface side even in a case of using the cross-linked hydrophilic coating agent.

This object is solved by a guidewire having the features of claim 1.

A guidewire according to the invention includes a core shaft, a coiled body including coils of strand wound around an outer circumference of the core shaft, and a layer of hydrophilic coating agent coating at least a part of the coiled body, wherein the hydrophilic coating agent is cross-linked by a cross-linking agent, and the degree of cross-linking of the hydrophilic coating agent by the cross-linking agent decreases in a radial outward direction from a side of the coiled body toward an outer surface side of the layer of hydrophilic coating agent.

It is to be noted that each coil of strand of the coils of strand forming the coiled body means a 360° turn or winding of the strand.

Advantageous embodiments are subject matter of dependent claims.

In one embodiment the layer of hydrophilic coating agent may form a single layer of hydrophilic coating agent. In another embodiment the layer of hydrophilic coating agent may form an inner or lower layer where the hydrophilic coating agent is cross-linked by the cross-linking agent so that the degree of cross-lining decreases in a radial outward direction thereof, and the inner or lower layer may be coated by an outer or upper layer of a hydrophilic coating agent containing no cross-linking agent.

### Brief Description of Drawings

The foregoing and other objects, features, aspects and advantages of the invention will become more apparent from the following detailed description when taken in conjunction with the accompanying drawings.
Fig. 1 is a vertical cross-sectional view showing a guidewire according to a first embodiment of the present invention.
Fig. 2 is a partially enlarged view of coils of strand constituting a coiled body and a hydrophilic coating agent coated on their surfaces in the first embodiment.
Fig. 3 is a partially enlarged view showing a modification example of the first embodiment.
Fig. 4 is a vertical cross-sectional view showing a guidewire according to a second embodiment of the present invention.
Fig. 5 is a partially enlarged view of coils of strand constituting a coiled body and a hydrophilic coating agent coated on their surfaces in the second embodiment.
Fig. 6 is a partially enlarged view showing a modification example of the second embodiment.

### Description of Embodiments

Preferred embodiments of the present invention will be described below with reference to the accompanying drawings, in which like reference characters designate similar or identical parts throughout the several views thereof.
<1> A guidewire according to a first aspect includes a core shaft, a coiled body including coils of strand wound around an outer circumference of the core shaft, and a layer of hydrophilic coating agent coating at least a part of the coiled body, wherein the hydrophilic coating agent is cross-linked by a cross-linking agent, and the degree of cross-linking of the hydrophilic coating agent by the cross-linking agent decreases in a radial outward direction from a side of the coiled body toward an outer surface side of the layer of hydrophilic coating agent.
<2> Also, a guidewire according to a second aspect includes a core shaft, a coiled body including coils of strand wound around an outer circumference of the core shaft, and a hydrophilic coating agent coating at least a part of the coiled body, wherein the hydrophilic coating agent has an inner or lower layer cross-linked by a cross-linking agent and an outer or upper layer coating the lower layer and containing no cross-linlcing agent, and the degree of cross-linking of the lower layer by the cross-linking agent decreases in a radial outward direction from a side of the coiled body toward a side of the upper layer.
<3> Further, a guidewire according to a third aspect is one in which, in the guidewire according to the second aspect, the lower layer of the hydrophilic coating agent contains a nonionic hydrophilic polymer while the upper layer of the hydrophilic coating agent contains at least one kind of polymer having an ionic functional group.
<1> As described above, in the guidewire according to the first aspect, the hydrophilic coating agent is cross-linked by the cross-linking agent. The degree of cross-linking of the hydrophilic coating agent by the cross-linking agent decreases from the side of the coiled body toward the outer surface side of the layer of hydrophilic coating agent. Thus, the degree of cross-linking of the hydrophilic coating agent is high on the side of the coiled body and is low on the outer surface side of the layer of hydrophilic coating agent. Accordingly, flexibility of the hydrophilic coating agent on the outer surface side of the layer of hydrophilic coating agent can be secured. Consequently, generation of cracks can be prevented, and lubricity of the hydrophilic coating agent can be secured.
<2> As described above, in the guidewire according to the second aspect, the hydrophilic coating agent includes two layers: the upper layer and the lower layer. The lower layer is cross-linked so that the degree of cross-linking by the cross-linking agent decreases in a radial outward direction from the side of the coiled body toward the side of the upper layer. On the other hand, the upper layer contains no cross-linking agent. In this configuration, strength of the lower layer is maintained while flexibility of the upper portion of the lower layer is secured. Also, decrease in the amount of the hydrophilic functional groups in the upper layer due to a cross-linking agent does not occur. Accordingly, since flexibility of the hydrophilic coating agent on the outer surface side can be secured, cracks can be prevented, and lubricity can be heightened.
<3> The guidewire according to the third aspect is one in which, in the guidewire according to the second aspect, the lower layer contains a nonionic hydrophilic polymer while the upper layer contains at least one kind of polymer having an ionic functional group. Since the lower layer contains a nonionic hydrophilic polymer and is cross-linked, the degree of swelling of the lower layer can be lowered when the hydrophilic coating agent comes into contact with a fluid, a body fluid or the like. Thus, strength of the lower layer can be heightened while flexibility of the upper portion of the lower layer can be secured. Also, since the upper layer contains at least one kind of polymer having an ionic functional group and is not cross-linked, the upper layer is swollen sufficiently upon contact with a fluid or a body fluid. Thus, flexibility and lubricity of the upper layer can be further improved.

It is to be noted that cross-linking means chemical cross-linking by a cross-linking agent.

Hereinafter, a guidewire according to a first embodiment of the present invention will be described with reference to the drawings.

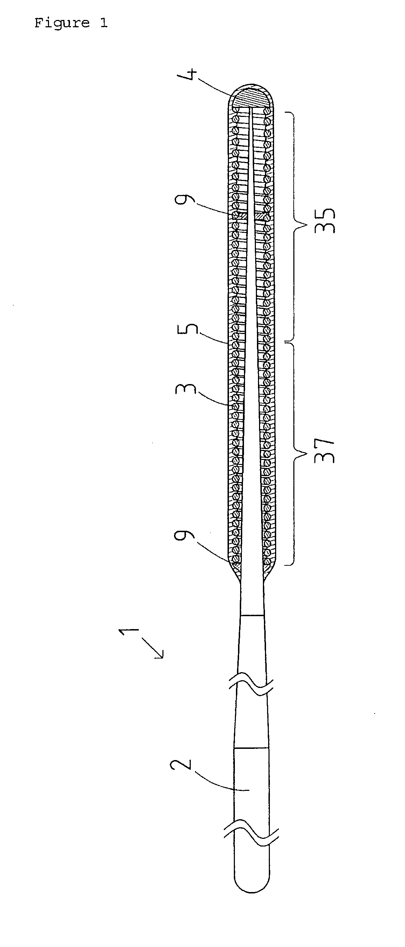

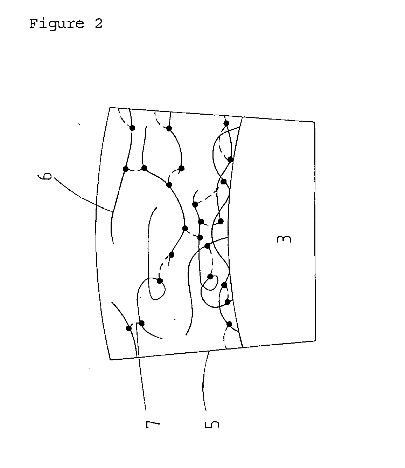

Fig. 1 is a vertical cross-sectional view showing the guidewire according to the first embodiment of the present invention, and Fig. 2 schematically shows a hydrophilic coating agent 5 by enlarging coils of strand of a coiled body 3 coated with the hydrophilic coating agent 5 in Fig. 1.

It is to be noted that, in Fig. 1, the left side is referred to as "a proximal side," and the right side is referred to as "a front side" for convenience of explanation.

Also, in Fig. 1, the guidewire is shortened in the longitudinal direction to show the entirety schematically for ease of understanding. Accordingly, the scale ratio of the entire guidewire shown in the drawings differs from the actual one.

In Fig. 1, a guidewire 1 has a core shaft 2 tapered toward the front end and a coiled body 3 covering the tip portion of the core shaft 2. The front end of the core shaft 2 and the front end of the coiled body 3 are fixed at a most distal portion 4. Also, the core shaft 2 and the coiled body 3 are fixed at brazed portions 9 (two locations) at positions on the proximal side from the most distal portion 4.

The outer circumference of the most distal portion 4 and the coiled body 3 of the guidewire 1 is coated with the hydrophilic coating agent 5.

A material constituting the core shaft 2 is not particularly limited. The material constituting the core shaft 2 can be selected from a stainless steel alloy, a super elastic alloy, a cobalt alloy, a piano wire, and tungsten, for example.

A material constituting the coiled body 3 fixed at the tip portion of the core shaft 2 can be selected from radiopaque metals such as platinum, gold, and tungsten and radiolucent metals such as a stainless steel alloy, a super elastic alloy, a cobalt alloy, and a piano wire, for example.

Examples of a material constituting the most distal portion 4 fixing the front end of the core shaft 2 and the front end of the coiled body 3 and the brazed portions 9 include an aluminum alloy brazing material, a silver brazing material, a gold brazing material, zinc, an Sn-Pb alloy, a Pb-Ag alloy, and an Sn-Ag alloy.

The hydrophilic coating agent 5 coating the coiled body 3 and the most distal portion 4 contains a hydrophilic polymer 6 as shown in Fig. 2. Also, to this hydrophilic coating agent 5 is added a cross-linking agent 7 cross-linking the hydrophilic polymer 6.

A component material for the hydrophilic polymer 6 may be any hydrophilic polymer as long as it can react with the cross-linking agent. Examples of the hydrophilic polymer include nonionic hydrophilic polymers such as polyvinyl alcohol, polyacrylamide, polymethylacrylamide, poly (2-hydroxyethyl methacrylate), and poly (N-hydroxyethyl acrylamide), anionic hydrophilic polymers such as polyacrylic acid, polymethacrylic acid, polymaleic acid, carboxymethyl cellulose, hyaluronic acid, and poly (2-acrylamide-2-methylpropanesulfonic acid), and cationic hydrophilic polymers such as polyethyleneimine, polyallylamine, and polyvinylamine. Also, plural kinds of these hydrophilic polymers may be used as the component materials for the hydrophilic polymer 6. It is to be noted that, in a case of using plural kinds of ionic functional groups as the component materials for the hydrophilic polymer 6, it is preferable to prevent an ion complex from being formed. Thus, in the case of using ionic functional groups, it is preferable to use only either anionic or cationic functional groups.

Among them, hyaluronic acid is an anionic hydrophilic polymer having a high molecular weight. Thus, the hyaluronic acid can maintain excellent strength even after the cross-linking and has excellent hydrophilicity. Accordingly, by using the hyaluronic acid, excellent lubricity at the time of contact with a fluid or a body fluid can be given to the hydrophilic polymer 6.

As a material for the cross-linking agent 7, a compound that reacts with a functional group of the hydrophilic polymer and cross-links the polymers can be used. Examples of the material for the cross-linking agent 7 include carbodiimide, water-soluble carbodiimide, an epoxy-based cross-linking agent, a polyvalent hydrazide-based cross-linking agent, a polyvalent aldehyde-based cross-linking agent, and a polyvalent aziridine-based cross-linking agent.

Among them, it is preferable to use a water-soluble cross-linking agent (e.g., water-soluble carbodiimide). Since a target for cross-linking is the hydrophilic polymer 6, such a cross-linking agent can be dissolved in a common solvent in the case of forming the hydrophilic coating agent 5.

Meanwhile, examples of the functional group possessed by the hydrophilic polymer include a hydroxyl group, a carboxyl group, an acid anhydride group, an amino group, and an amide group. The functional group is not limited to these but can be any functional group as long as it can react with the cross-linking agent 7.

Also, when the coiled body 3 and the like are coated with the hydrophilic coating agent 5, the hydrophilic coating agent 5 comes into contact with a hydrophobic air interface. Thus, in a case where the cross-linking agent 7 is hydrophilic, it moves to the side of the coiled body 3. Accordingly, the degree of cross-linking of the hydrophilic coating agent 5 existing on the side of the coiled body 3 can be heightened.

In this manner, strength of the hydrophilic coating agent 5 on the side of the coiled body 3 is increased. In addition, since flexibility of the hydrophilic coating agent 5 on the surface side is heightened, cracks at the hydrophilic coating agent 5 can be prevented. Further, since the amount of cross-links is small on the surface side of the hydrophilic coating agent 5, decrease in the amount of the hydrophilic functional groups as starting points of the cross-linking reactions can be suppressed. Accordingly, lubricity of the hydrophilic coating agent 5 on the surface side can be secured.

Next, the cross-linked hydrophilic coating agent 5 coating the outer circumference of the most distal portion 4 and the coiled body 3 will be described.

As shown in Fig. 2, the hydrophilic coating agent 5 contains the hydrophilic polymer 6. Also, to the hydrophilic coating agent 5 is added the cross-linking agent 7 cross-linking the hydrophilic polymer 6. The degree of cross-linking of the hydrophilic coating agent 5 decreases from the side of the coiled body 3 toward the surface side of the hydrophilic coating agent 5. Such a mechanism for decreasing in the degree of cross-linking will be described below together with a process for coating the hydrophilic coating agent 5.

The hydrophilic polymer 6 and the cross-linking agent 7 constituting the hydrophilic coating agent 5 are dissolved in their good solvent. The most distal portion 4 and the coiled body 3 of the guidewire 1 are immersed in the solution in which the hydrophilic coating agent has been dissolved. Thereafter, the guidewire 1 is pulled up and dried (the solvent is evaporated) while a reaction by the cross-linking agent 7 is accelerated. In this drying process, the hydrophilic coating agent 5 is dried from the surface side. Thus, in the inside of the hydrophilic coating agent 5, the solvent and the unreacted cross-linking agent remain. The remaining cross-linking agent reacts with the hydrophilic polymer existing near the coiled body 3 to cause the cross-linking to progress. In a state where the solvent has been evaporated completely, the guidewire 1 is in a state where the degree of cross-linking decreases from the side of the coiled body 3 toward the surface side of the hydrophilic coating agent 5.

When the guidewire 1 coated with the hydrophilic coating agent 5 prepared as above comes into contact with a fluid or a body fluid, the hydrophilic coating agent 5 is swollen and exerts lubricity. The degree of cross-linking of the hydrophilic coating agent 5 is higher on the side of the coiled body 3 than on the surface side. Thus, strength of the hydrophilic coating agent 5 on the side of the coiled body 3 is secured. Also, flexibility of the hydrophilic coating agent 5 on the surface side is secured. Thus, cracks at the hydrophilic coating agent 5 can be prevented, and lubricity of the hydrophilic coating agent 5 on the surface side can be secured.

The good solvent used in the first embodiment only needs to be a highly polar solvent. The good solvent can be selected from water, methanol, ethanol, N-propanol, acetonitrile, isopropyl alcohol, dimethyl sulfoxide, dimethyl acetamide, dimethyl formamide, and N-methyl-2-pyrrolidone, for example.

In coating the surface of the coiled body 3 with the hydrophilic coating agent 5, the surface of the coiled body 3 may be irradiated with ultraviolet or plasma before being coated with the hydrophilic coating agent 5 to activate the surface of the coiled body 3.

This enables bonding strength between the hydrophilic coating agent 5 and the coiled body 3 to be heightened. Also, on the surface of the coiled body 3 activated by ultraviolet or plasma, more hydrophilic polymers 6 in the hydrophilic coating agent 5 gather on the side of the coiled body 3. This can give variety to the degree of cross-linking in the hydrophilic coating agent 5.

Consequently, strength of the hydrophilic coating agent 5 on the side of the coiled body 3 is heightened while flexibility on the surface side is secured. Thus, cracks at the hydrophilic coating agent 5 can be prevented, and lubricity of the hydrophilic coating agent 5 on the surface side can be secured.

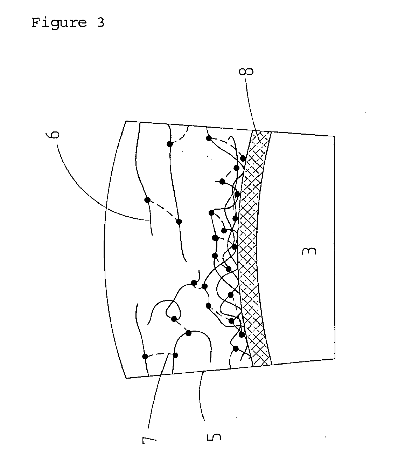

Also, in a modification example of the first embodiment, a coupling layer 8 made of a compound having a coupling characteristic with both the coiled body 3 and the hydrophilic polymer 6 may be provided between the hydrophilic coating agent 5 and the coiled body 3, as shown in Fig. 3.

A material forming the coupling layer 8 only needs to be a compound having a bonding characteristic with a metal and a hydrophilic polymer. Examples of such a compound include an epoxy-based resin, an isocyanate-based resin, a phenol-based resin, a resin containing phosphoric acid on a monomer unit such as poly (ethyleneglycol methacrylate phosphate), a silane coupling agent, a titanate coupling agent, and a DLC film.

Among them, it is preferable to use the resin containing phosphoric acid on a monomer unit as the coupling layer 8. The resin containing phosphoric acid on a monomer unit is excellent in coupling with a metal and a hydrophilic polymer 6. Also, the resin itself is hydrophilic. Accordingly, affinity of the hydrophilic polymer 6 for the cross-linking agent 7 can be heightened by the coupling layer 8. Consequently, coupling of the hydrophilic coating agent 5 with the coiled body 3 can be further heightened.

Also, when the coupling layer 8 is provided, more hydrophilic polymers 6 in the hydrophilic coating agent 5 gather on the side of the coiled body 3. Thus, the degree of cross-linking on the side of the coiled body 3 is further heightened while the degree of cross-linking of the hydrophilic coating agent 5 on the surface side is lowered.

By providing such a coupling layer 8, strength of the hydrophilic coating agent 5 on the side of the coiled body 3 can be further heightened. Also, by the coupling layer 8, flexibility of the hydrophilic coating agent 5 on the surface side is secured. Thus, cracks at the hydrophilic coating agent 5 can be prevented, and lubricity of the hydrophilic coating agent 5 on the surface side can be secured.

Meanwhile, the coiled body 3 may be irradiated with the aforementioned ultraviolet or plasma before being provided with the coupling layer 8 to heighten bonding strength between the coiled body 3 and the coupling layer 8.

Also, by using a compound that reacts with the cross-linking agent 7 as a material for the coupling layer 8, the coupling layers 8 can be cross-linked. By doing so, the coupling layer 8 is strengthened. This can strengthen coupling of the coiled body 3 with the hydrophilic coating agent 5.

Further, the coupling layer 8 and the hydrophilic coating agent 5 can be cross-linked by the cross-linking agent 7. This can strengthen coupling of the coiled body 3 with the hydrophilic coating agent 5.

The thickness of the hydrophilic coating agent 5 in the first embodiment and the modification example of the first embodiment may be changed in accordance with desired characteristics. Also, the thickness of the hydrophilic coating agent 5 may be changed in a longitudinal direction of the coiled body 3.

Also, in the first embodiment and the modification example of the first embodiment, the entirety of the most distal portion 4 and the coiled body 3 is coated with the hydrophilic coating agent 5. However, the coating range of the hydrophilic coating agent 5 is not limited to this but may be only the coiled body 3 or only a front side portion 35 or a proximal side portion 37 of the coiled body 3.

Next, a guidewire according to a second embodiment of the present invention will be described with reference to the drawings.

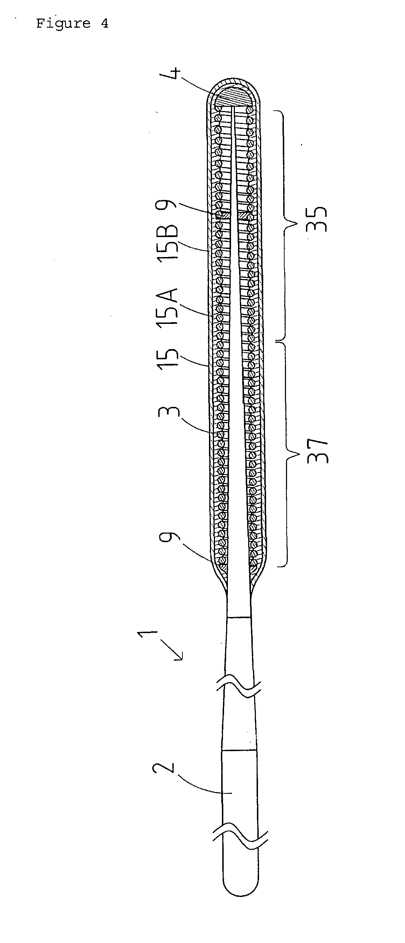

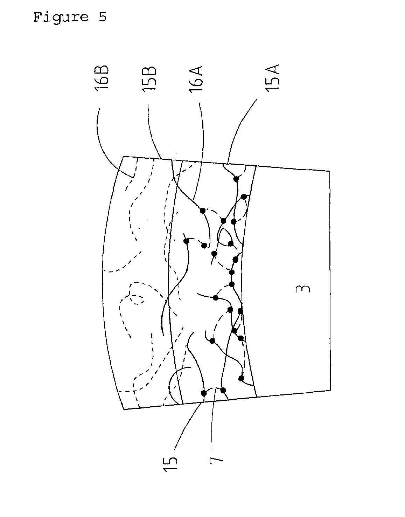

The second embodiment is a guidewire 1 coated with a hydrophilic coating agent 15 including two layers. This will be described with reference to Figs. 4 and 5 mainly on the difference from the first embodiment.

Fig. 4 is a vertical cross-sectional view showing the guidewire according to the second embodiment of the present invention, and Fig. 5 schematically shows the hydrophilic coating agent 15 by enlarging coils of strand of the coiled body 3 coated with the hydrophilic coating agent 15 in Fig. 4. It is to be noted that, in Fig. 4, the guidewire is shortened in the longitudinal direction to show the entirety schematically for ease of understanding in a similar manner to Fig. 1. Accordingly, the scale ratio of the entire guidewire shown in the drawings differs from the actual one.

The hydrophilic coating agent 15 includes a lower layer 15A and an upper layer 15B.

The lower layer 15A is formed by a hydrophilic polymer 16A and a cross-linking agent 7. The degree of cross-linking of the lower layer 15A decreases in a direction from the side of the coiled body 3 toward the side of the upper layer of the hydrophilic coating agent 15.

The upper layer 15B is formed by a hydrophilic polymer 16B. In this upper layer 15B, no cross-linking by a cross-linking agent is formed.

In this configuration, strength of the lower layer 15A on the side of the coiled body 3 is heightened. Thus, flexibility of the upper layer portion of the lower layer 15A can be secured. Also, in the upper layer 15B, no cross-linking by a cross-linking agent is formed. Thus, the upper layer 15B can obtain high flexibility. Accordingly, cracks at the hydrophilic coating agent 15 can be prevented. In addition, in the upper layer 15B, decrease in the amount of the hydrophilic functional groups by a cross-linking reaction does not occur. Since the upper layer 15B can greatly exert hydrophilicity, it can obtain excellent lubricity.

In this configuration, in which the surface of the lower layer 15A having the low degree of cross-linking is coated with the upper layer 15B, the upper layer portion of the lower layer 15A is swollen by a good solvent of the upper layer 15B in which the hydrophilic polymer has been dissolved. The hydrophilic polymer of the upper layer 15B goes into the swollen portion of the lower layer 15A. In this manner, a mixed layer including the lower layer 15A and the upper layer 15B is formed. Therefore, the hydrophilic polymer forming the upper layer 15B and the hydrophilic polymer forming the lower layer 15A intertwine with each other. This increases a coupling force between the lower layer 15A and the upper layer 15B, and thus durability of the hydrophilic coating agent can be improved.

A material for the hydrophilic polymer 16A constituting the lower layer 15A may be any hydrophilic polymer as long as it can react with the cross-linking agent. Examples of the hydrophilic polymer include nonionic hydrophilic polymers such as polyvinyl alcohol, polyacrylamide, polymethylacrylamide, poly (2-hydroxyethyl methacrylate), and poly (N-hydroxyethyl acrylamide), anionic hydrophilic polymers such as polyacrylic acid, polymethacrylic acid, polymaleic acid, carboxymethyl cellulose, hyaluronic acid, and poly (2-acrylamide-2-methylpropanesulfonic acid), and cationic hydrophilic polymers such as polyethyleneimine, polyallylamine, and polyvinylamine. Also, plural kinds of these hydrophilic polymers may be used. It is to be noted that, in a case of using plural kinds of ionic functional groups as the materials for the hydrophilic polymer 16A, it is preferable to prevent an ion complex from being formed. Thus, in the case of using ionic functional groups, it is preferable to use only either anionic or cationic functional groups.

Among them, a preferable material for the hydrophilic polymer 16A constituting the lower layer 15A is a nonionic hydrophilic polymer. The nonionic hydrophilic polymer has a lower degree of swelling at the time of absorbing a fluid than the polyanionic and polycationic hydrophilic polymers. Thus, in the nonionic hydrophilic polymer, hydrophilic polymer chains exist relatively close to one another. Accordingly, the nonionic hydrophilic polymer can be cross-linked more densely than the anionic and cationic hydrophilic polymers.

By using the nonionic hydrophilic polymer as the material for the lower layer 15A, dense cross-linking is formed in the lower layer 15A. Thus, as described above, the degree of cross-linking of the lower layer 15A decreases from the side of the coiled body 3 toward the side of the upper layer 15B. Accordingly, coupling strength on the side of the coiled body 3 can be further heightened.

Meanwhile, the cross-linking agent 7 used for the lower layer 15A may be the same as the cross-linking agent 7 used in the first embodiment.

As a material for the hydrophilic polymer 16B constituting the upper layer 15B, the same material as that for the hydrophilic polymer constituting the lower layer 15A may be used. The hydrophilic polymer 16B preferably contains at least one kind of hydrophilic polymer having an ionic functional group such as a polyanionic or polycationic functional group. The hydrophilic polymer having an ionic functional group has a higher degree of swelling at the time of absorbing a fluid than the nonionic hydrophilic polymer. Accordingly, by using the hydrophilic polymer having an ionic functional group for the upper layer 15B, flexibility and lubricity of the upper layer 15B can be improved.

In the case of using the hydrophilic polymer having an ionic functional group as the material for the upper layer 15B, when the upper layer 15B comes into contact with a fluid or a body fluid and exerts its lubricity, the polymer chains having ionic functional groups existing in the upper layer 15B are to be dispersed by electrostatic repulsion of the ionic functional groups themselves. Accordingly, flexibility of the upper layer 15B can be improved. Also, in this case, a narrow space is generated between the hydrophilic polymer chains that are to be dispersed, and water molecules flow into this space. Accordingly, lubricity of the upper layer 15B can be heightened.

In coating the surface of the coiled body 3 of the guidewire 1 with the hydrophilic coating agent 15 having the lower layer 15A, the surface of the coiled body 3 may be irradiated with ultraviolet or plasma for activation before coating.

This enables bonding strength between the lower layer 15A and the coiled body 3 to be heightened. More hydrophilic polymers 16A in the lower layer 15A gather on the side of the surface of the coiled body 3 activated by irradiation with ultraviolet or plasma. This can give variety to the degree of cross-linking in the lower layer 15A.

Consequently, strength of the hydrophilic coating agent 15 on the side of the coiled body 3 can be heightened while flexibility of the hydrophilic coating agent 15 on the surface side can be secured. Thus, cracks at the hydrophilic coating agent 15 can be prevented.

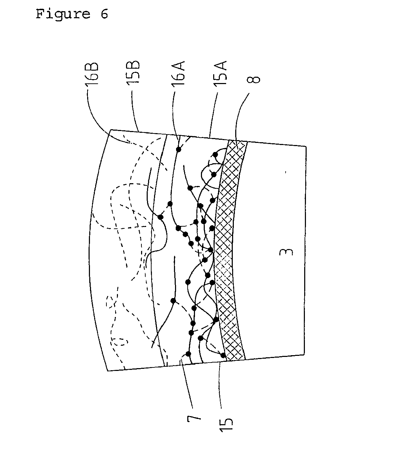

A modification example of the second embodiment is shown in Fig. 6. As in the modification example, a coupling layer 8 having a coupling characteristic with both the lower layer 15A and the coiled body 3 may be provided between the lower layer 15A and the coiled body 3.

As a material for forming this coupling layer 8, the same material as that in the first embodiment can be used.

By providing the coupling layer 8, strength of the lower layer 15A on the side of the coiled body 3 can be further heightened. Also, flexibility of the lower layer 15A on the upper layer portion side is secured. Thus, cracks at the hydrophilic coating agent 15 can be prevented, and lubricity of the hydrophilic coating agent 15 on the surface side can be secured.

Meanwhile, the coiled body 3 may be irradiated with the aforementioned ultraviolet or plasma before being coated with the coupling layer 8 to heighten bonding strength between the coiled body 3 and the coupling layer 8.

Also, the coupling layer 8 can be cross-linked by the cross-linking agent 7. By doing so, the coupling layer 8 is strengthened. This can strengthen coupling of the coiled body 3 with the lower layer 15A.

Further, the coupling layer 8 and the lower layer 15A can be cross-linked by the cross-linking agent 7. This can further strengthen the lower layer 15A on the side of the coiled body 3.

A process for coating the surfaces of the coiled body 3 and the most distal portion 4 of the guidewire 1 with the lower layer 15A and the upper layer 15B will be described below.

First, the coiled body 3 and the most distal portion 4 of the guidewire 1 are irradiated with ultraviolet or plasma. This activates the metal surfaces of the coiled body 3 and the most distal portion 4.

Next, the material forming the coupling layer 8 is dissolved in a solvent. The surfaces of the activated coiled body 3 and most distal portion 4 are immersed in this solution to coat the surfaces of the coiled body 3 and the most distal portion 4 with the solution. Thereafter, the surfaces of the coiled body 3 and the most distal portion 4 are dried to form the coupling layer 8.

Meanwhile, as the solvent in which the coupling layer 8 is dissolved, the highly polar solvent used in the first embodiment can be used. However, it is not limited to this but can be any good solvent for the material for the coupling layer 8.

Subsequently, the hydrophilic polymer 16A and the cross-linking agent 7 forming the lower layer 15A of the hydrophilic coating agent 15 are dissolved in their good solvent. The portion provided with the coupling layer 8 is immersed in the solution in which the material for the lower layer 15A has been dissolved, to coat this portion with the solution. Thereafter, this portion is dried to form the lower layer 15A, in which the degree of cross-linking decreases in a direction from the side of the coiled body 3 toward the surface side (the side of the upper layer 15B).

Lastly, the hydrophilic polymer 16B forming the upper layer 15B of the hydrophilic coating agent 15 is dissolved in a good solvent. The portion provided with the lower layer 15A is immersed in the solution in which the material for the upper layer 15B has been dissolved, to coat this portion with the solution. Thereafter, this portion is dried to form the upper layer 15B.

In this manner, the hydrophilic coating agent 15 in which the degree of cross-linking in the lower layer 15A decreases from the side of the coiled body 3 toward the side of the upper layer 15B can be prepared. Also, the hydrophilic coating agent 15 can be prepared by any of various known processes instead of the aforementioned immersion process.

Meanwhile, the good solvents for the lower layer 15A and the upper layer 15B used in the second embodiment only need to be highly polar solvents. The good solvents can be selected from water, methanol, ethanol, N-propanol, acetonitrile, isopropyl alcohol, dimethyl sulfoxide, dimethyl acetamide, dimethyl formamide, and N-methyl-2-pyrrolidone, for example.

The thickness of the hydrophilic coating agent 15 in the second embodiment may be changed in accordance with characteristics desired in the product. For example, the thickness of the hydrophilic coating agent 15 may be changed at the front side portion 35 and the proximal side portion 37 of the coiled body 3.

Also, in the second embodiment, the entirety of the most distal portion 4 and the coiled body 3 is coated with the hydrophilic coating agent 15. However, the coating range of the hydrophilic coating agent 15 is not limited to this but may be only the coiled body 3 or only the front side portion 35 or the proximal side portion 37 of the coiled body 3.

Also, the front side portion 35 of the coiled body 3 may be coated with the hydrophilic coating agent 15 in the second embodiment while the proximal side portion 37 of the coiled body 3 may be coated with the hydrophilic coating agent 5 in the first embodiment.

As for the second embodiment, other modification examples can be assumed, and the second embodiment may be altered or improved in various ways by those skilled in the art without departing from the spirit and scope of the present invention.

### [Examples]

Hereinafter, specific examples of the present invention will be described.

### (Example 1)

First, 2 g of polyvinyl alcohol and 1 g of water-soluble carbodiimide were dissolved in 106 ml of dimethyl formamide as a solvent to prepare a solution of a hydrophilic coating agent (Solution A).

Next, the most distal portion made of Sn-Ag and the coiled body made of high corrosion resistance stainless steel (SUS316) of the guidewire shown in Fig. 1 were immersed in Solution A.

Next, the most distal portion and the coiled body of the above guidewire were dried in a drying furnace at 120°C for 2 hours. By doing so, dimethyl formamide as a solvent was removed, and a cross-linking reaction by water-soluble carbodiimide was accelerated.

In this manner, the guidewire shown in Fig. 1 coated with the hydrophilic coating agent having a thickness of 1 µm in which the degree of cross-linking decreases in a direction from the side of the coiled body toward the surface side was obtained.

### (Example 2)

First, 2 g of polymer including acid phosphoxy methacrylate containing a phosphoric group on a monomer unit (product name: Phosmer M, produced by Uni-Chemical Co., Ltd.) was dissolved in 100 ml of water as a solvent to prepare Solution B containing a material for a coupling layer.

Next, 0.5 g of sodium hyaluronate and 1 g of water-soluble carbodiimide were dissolved in 100 ml of water as a solvent to prepare a solution of a hydrophilic coating agent (Solution C).

Next, the most distal portion and the coiled body of the same guidewire as that in Example 1 were irradiated with 172 nm UV in the atmosphere for 5 minutes with use of a vacuum ultraviolet exposure system (manufactured by Ushio Inc.).

Next, the portion irradiated with UV was immersed in Solution B. Thereafter, the portion was dried in the drying furnace at 120°C for 2 hours to remove the solvent. By doing so, the coupling layer was formed on the surfaces of the coiled body and the most distal portion.

Next, the portion on which the coupling layer had been formed was immersed in Solution C. Thereafter, the portion was dried in the drying furnace at 120°C for 3 hours to accelerate a cross-linking reaction at the portion. In this manner, the guidewire having the hydrophilic coating agent shown in Fig. 3 coated with the hydrophilic coating agent having a thickness of 3 µm in which the degree of cross-linking decreases in a direction from the side of the coiled body toward the surface side was obtained.

### (Example 3)

First, Solution B containing the same material as that in Example 2 was prepared.

Next, 2 g of polyvinyl alcohol and 1 g of water-soluble carbodiimide were dissolved in 100 ml of water as a solvent to prepare Solution D of a hydrophilic coating agent as a lower layer.

Next, 0.5 g of sodium hyaluronate and 3 g of carboxymethyl cellulose were dissolved in 100 ml of water as a solvent to prepare Solution E of a hydrophilic coating agent as an upper layer.

Next, the most distal portion and the coiled body of the same guidewire as that in Example 1 were irradiated with UV under the same conditions as those in Example 2.

Next, the portion irradiated with UV was immersed in Solution B containing the material for the coupling layer. Thereafter, the portion was dried in the drying furnace at 120°C for 30 minutes to remove the solvent. By doing so, the coupling layer was formed on the surfaces of the coiled body and the most distal portion.

Next, the portion on which the coupling layer had been formed was immersed in Solution D containing the material for the hydrophilic coating agent as the lower layer. Thereafter, the portion was dried in the drying furnace at 120°C for 2 hours to accelerate a cross-linking reaction at the portion. In this manner, the lower layer in which the degree of cross-linking decreases in a direction from the side of the coiled body toward the surface side was formed.

Next, the portion on which the lower layer had been formed was immersed in Solution E containing the material for the hydrophilic coating agent as the upper layer. Thereafter, the portion was dried in the drying furnace at 120°C for 3 hours. In this manner, the guidewire having the hydrophilic coating agent shown in Fig. 6 coated with the hydrophilic coating agent having a thickness of 4 µm including two layers was obtained.

While the invention has been shown and described in detail, the foregoing description is in all aspects illustrative and not restrictive.

### Reference Signs List

- 1: guidewire
- 2: core shaft
- 3: coiled body
- 4: most distal portion
- 5: hydrophilic coating agent
- 15: hydrophilic coating agent
- 15A: lower layer (hydrophilic coating agent)
- 15B: upper layer (hydrophilic coating agent)
- 6: hydrophilic polymer
- 16A: hydrophilic polymer (lower layer)
- 16B: hydrophilic polymer (upper layer)
- 7: cross-linking agent
- 8: coupling layer

## Claims

1. A guidewire (1) comprising:
a core shaft (2);
a coiled body (3) including coils of strand wound around an outer circumference of the core shaft (2); and
a layer of hydrophilic coating agent (5; 15) coating at least a part of the coiled body (3),
wherein the hydrophilic coating agent (5; 15) is cross-linked by a cross-linking agent (7), **characterized in that**
the degree of cross-linking of the hydrophilic coating agent (5; 15) by the cross-linking agent (7) decreases in a radial outward direction from a side of the coiled body (3) toward an outer surface side of the layer of hydrophilic coating agent (5; 15).

2. A guidewire (1) according to claim 1, comprising a single layer of hydrophilic coating agent (5).

3. A guidewire (1) according to claim 1, wherein the layer of hydrophilic coating agent (15) forms a lower layer (15A) where the hydrophilic coating agent (15) is cross-linked by the cross-linking agent (7), and wherein the lower layer (15A) is coated by an upper layer (15B) of hydrophilic coating agent (15) containing no cross-linking agent.

4. The guidewire (1) according to claim 3, wherein the lower layer (15A) of the hydrophilic coating agent (15) contains a nonionic hydrophilic polymer while the upper layer (15B) of the hydrophilic coating agent (15) contains at least one kind of polymer having an ionic functional group.

## Patentansprüche

1. Führungsdraht (1) mit:
einem Kernschaft (2);
einem gewickelten Körper (3), der um einen äußeren Umfang des Kernschafts (2) gewundene Drahtwicklungen umfasst; und
einer Schicht eines hydrophilen Ummantelungsmittels (5; 15), das wenigstens einen Teil des gewickelten Körpers (3) ummantelt,
wobei das hydrophile Ummantelungsmittel (5; 15) durch ein Vernetzungsmittel (7) vernetzt ist, **dadurch gekennzeichnet, dass**
der Grad der Vernetzung des hydrophilen Ummantelungsmittels (5; 15) durch das Vernetzungsmittel (7) von einer Seite des gewickelten Körpers (3) radial nach außen zur Seite einer äußeren Oberfläche der Schicht des hydrophilen Ummantelungsmittels (5; 15) abnimmt.

2. Führungsdraht (1) nach Anspruch 1, mit einer einzigen Schicht des hydrophilen Ummantelungsmittels (5).

3. Führungsdraht (1) nach Anspruch 1, wobei die Schicht des hydrophilen Ummantelungsmittels (15) eine untere Schicht (15A) bildet, wo das hydrophile Ummantelungsmittel (15) durch das Vernetzungsmittel (7) vernetzt ist, und wobei die untere Schicht (15A) durch eine obere Schicht (15B) des hydrophilen Ummantelungsmittels (15), das kein Vernetzungsmittel enthält, beschichtet ist.

4. Führungsdraht (1) nach Anspruch 3, wobei die untere Schicht (15A) des hydrophilen Ummantelungsmittels (15) ein nicht-ionisches, hydrophiles Polymer enthält, während die obere Schicht (15B) des hydrophilen Ummantelungsmittels (15) wenigstens eine Polymerart enthält, die keine ionische funktionale Gruppe enthält.

## Revendications

1. Fil-guide (1) comprenant :
une broche d'enroulage (2) ;
un corps enroulé (3) incluant des bobines de fil entourées autour d'une circonférence externe de la broche d'enroulage (2) ; et
une couche d'agent de revêtement hydrophile (5 ; 15) revêtant au moins une partie du corps enroulé (3),
dans lequel l'agent de revêtement hydrophile (5 ; 15) est réticulé par un agent de réticulation (7), **caractérisé en ce que**
le degré de réticulation de l'agent de revêtement hydrophile (5 ; 15) par l'agent de réticulation (7) diminue dans une direction extérieure radiale depuis un côté du corps enroulé (3) vers un côté de surface externe de la couche d'agent de revêtement hydrophile (5 ; 15).

2. Fil-guide (1) selon la revendication 1, comprenant une seule couche d'agent de revêtement hydrophile (5).

3. Fil-guide (1) selon la revendication 1, dans lequel la couche d'agent de revêtement hydrophile (15) forme une couche inférieure (15A) où l'agent de revêtement hydrophile (15) est réticulé par l'agent de réticulation (7), et dans lequel la couche inférieure (15A) est revêtue par une couche supérieure (15B) d'agent de revêtement hydrophile (15) ne contenant aucun agent de réticulation.

4. Fil-guide (1) selon la revendication 3, dans lequel la couche inférieure (15A) de l'agent de revêtement hydrophile (15) contient un polymère hydrophile non ionique tandis que la couche supérieure (15B) de l'agent de revêtement hydrophile (15) contient au moins une sorte de polymère ayant un groupe fonctionnel ionique.
